# EUROPÄISCHE PATENTANMELDUNG

(11) **EP 0 597 520 A1**
(43) Veröffentlichungstag der Anmeldung: **18.05.1994**
(21) Anmeldenummer: 93203037.2
(22) Anmeldetag: 29.10.1993
(51) Int. Cl.: C07F 1/02, C07B 61/00

(54) **Pulverförmige, nicht pyrophore Lithiumalkylverbindung und Verfahren zu ihrer Herstellung**

(30) Priorität: 10.11.1992 DE 4237884
(71) Anmelder: METALLGESELLSCHAFT AG, D-60323 Frankfurt am Main (DE)
(72) Erfinder: Weiss, Wilfried, Dr., D-61440 Oberursel (DE); Emmel, Ute, D-65929 Frankfurt am Main (DE)

(57) **Zusammenfassung**

Die Erfindung betrifft eine pulverförmige, nicht pyrophore Lithiumalkylverbindung der allgemeinen Formel LiCH₃ · Xₙ, bei der x = ein Ether sowie n < 1 ist. Die Erfindung betrifft ferner ein Verfahren zur Herstellung dieser Verbindung.

## Beschreibung

Die Erfindung betrifft eine pulverförmige, nicht pyrophore Lithiumalkylverbindung.

Lithiumorganische Verbindungen spielen in der präparativen organischen Chemie eine bedeutende Rolle, um Lithium oder einen organischen Substituenten in eine organische Verbindung einzuführen. Die lithiumorganischen Verbindungen sind im allgemeinen luft- und feuchtigkeitsempfindlich und daher nur unter Vorsichtsmaßnahmen zu handhaben.

Der pyrophore Charakter der Lithiumorganyle hat dazu geführt, daß man ihre wichtigsten Vertreter (n-, s-, t-Butyllithium) als verdünnte Lösungen in Kohlenwasserstoffen auf den Markt bringt. Die aufgrund des hohen Dampfdruckes über der Lösung liegende Lösemittelgasschicht wirkt wie ein Schutzschild gegen atmosphärische Angriffe auf die aktive Lithiumverbindung.

Methyllithium und Ethyllithium sind im Gegensatz zu ihren höheren Homologen in gesättigten und ungesättigten Kohlenwasserstoffen nicht oder nur wenig löslich, als Feststoffe jedoch in hohem Maße pyrophor und daher schwierig handhabbar.

Die Synthese von Methyllithium erfolgt in an sich bekannter Weise. Methyllithium wird in etherhaltigen Lösemitteln durch Umsetzung von metallischem Lithium und Alkylhalogenid gemäß der Formel

RHal + 2 Li --> RLi + LiHal

hergestellt. Methyllithium besitzt die beste Löslichkeit in Diethylether, worin es zu etwa 5 bis 6 % löslich ist. Die Beschränkung auf Diethylether als Lösemittel schmälert jedoch den Einsatz von Methyllithium, weil viele Anwender den Gebrauch von Diethylether im industriellen Maßstab wegen des hohen Dampfdrucks und der Neigung zur Peroxidbildung scheuen und nach Möglichkeit zu umgehen versuchen. Außerdem sind etherische Lösungen von Lithiumorganylen nicht lagerstabil. Ether wie Tetrahydrofuran werden durch Methyllithium langsam gespalten und aus Diethylether fällt polymeres Methyllithium aus. Die Komplexe von Methyllithium mit Lithiumhalogeniden zeigen gelöst in Ethern aber eine bemerkenswerte Stabilität.

Aus der EP-PS 0 285 374 sind stabilisierte Lösungen von Lithiumorganylen bekannt, die neben Magnesiumorganylen noch eine Lewis Base, wie Tetrahydrofuran, enthalten. Allerdings enthalten diese Lösungen geringe Mengen an Lithiumorganylen und sind nur begrenzt lagerstabil.

Aus der DE-OS 38 15 166 sind nicht pyrophore, rieselfähige Synthesemittel für lithiumorganische Reaktionen bekannt, bei denen Methyllithium oder Ethyllithium zusammen mit Paraffinen auf Oxiden aus der Gruppe SiO₂, Al₂O₃, CaO oder synthetischen, wasserfreien Alumosilikaten aufgebracht werden. Der durchschnittliche Gehalt an Lithiumverbindungen in den Trägern liegt bei 20 bis 25 %.

Der Erfindung liegt die Aufgabe zugrunde, eine pulverförmige, nicht pyrophore Lithiumalkylverbindung bereitzustellen, die einen hohen Gehalt an Aktiv-Base besitzt, nicht pyrophor ist und sich für den Einsatz in den üblicherweise in der Chemie der metallorganischen Verbindungen verwendeten Lösemitteln eignet, wobei auf anorganische Träger verzichtet werden soll.

Erfindungsgemäß wird die Aufgabe dadurch gelöst, daß die Lithiumverbindung der allgemeinen Formel LiCH₃ · xₙ entspricht, bei der x = ein Ether sowie n < 1 ist.

Die pulverförmige, nicht pyrophore Lithiumalkylverbindung wird erhalten durch Verdünnen einer Lösung von Methyllithium in Diethylether mit Kohlenwasserstoffen um 10 bis 50 Vol.% und anschließendes Verdampfen der Lösemittel bis zur Ausfällung der Lithiumalkylverbindung.

Überraschenderweise ist das erhaltene Produkt, das einen Gehalt an Aktiv-Base von ca. 70 % hat, nicht pyrophor, d.h. es ist kurzzeitig in trockener Luft handhabbar. Bei Lagerung unter Luftzutritt findet eine Zersetzung statt, aber erst durch unmittelbaren Kontakt mit Wasser kommt es zur Entzündung der Verbindung.

In weiterer Ausgestaltung der Erfindung wird die pulverförmige, nicht pyrophore Lithiumalkylverbindung hergestellt, indem man zur gesättigten Lösung von Methyllithium in Diethylether und einem aromatischen Kohlenwasserstoff zusätzlich einen oberhalb 50°C siedenden aliphatischen Ether mit mindestens 5 C-Atomen zugibt und die Lösemittel bis zum Ausfallen der Lithiumverbindung verdampft. Der oberhalb 50°C siedende aliphatische Ether mit mindestens 5 C-Atomen ist vorzugsweise Methyl-t-butylether, Di-n-butylether, Diisopropylether oder 1,2-Dimethoxyethan. Als aromatischer Kohlenwasserstoff zum Verdünnen der Diethylether-Lösung des Methyllithiums werden Benzen, Toluen, Xylen oder ihre Mischungen eingesetzt. Eine vorteilhafte Variante des erfindungsgemäßen Verfahrens besteht in der Verdampfung der Lösemittel bei reduziertem Druck, insbesondere bei 0,1 bis 10 mbar, im Ölpumpenvakuum, weil Zersetzungsreaktionen dadurch stark vermindert werden.

Die pulverförmige, nicht pyrophore Lithiumalkylverbindung wird in vorteilhafter Weise unter Schutzgas abfiltriert und durch Waschen mit trockenen, inerten Lösemitteln, z.B. Kohlenwasserstoffen, gereinigt und im Vakuum getrocknet. Das Filtrat und das Destillat können in vorteilhafter Weise im Verfahren rezykliert werden.

Die erhaltenen Produkte sind als Synthesemittel für die Durchführung von lithiumorganischen Metallierungs- und/oder Alkylierungsreaktionen geeignet, da sie in hochkonzentrierter Form in die in der Chemie der metallorganischen Verbindungen verwendeten Lösemittel eingetragen und durch Zusatz von Diethylether oder Tetrahydrofuran in Lösung gebracht werden können. Weitere Vorteile sind, daß keine anorganischen Feststoffe in das Reaktionsgemisch eingetragen werden und daß der Transport sowie die Lagerung dieser Verbindungen vergleichsweise unproblematisch ist.

Die Erfindung wird mit den nachfolgenden Beispielen erläutert.

### Beispiel 1

100 mmol (13 g) Di-n-butylether werden in 50 ml Toluen vorgelegt und innerhalb von 10 min mit 100 mmol etherischer Methyllithium-Lösung (60 ml) versetzt. Man erhält eine klare Lösung, wobei eine Temperaturerhöhung um 2°C beobachtet wird. Bis zu einer Siedetemperatur von 60°C wird das Lösemittel entfernt, wobei das Produkt als weißer Niederschlag ausfällt, der abfiltriert und im Ölpumpenvakuum getrocknet wird. Es werden 1,95 g weißes Pulver mit einer Alkalität von 40,6 mmol/g erhalten. Das erhaltene Produkt hat die Formel CH₃Li · [(C₄H₉)₂O]_{0,02} und ist an der Luft nicht brennbar.

### Beispiel 2

50 mmol (4,5 g) 1,2-Dimethoxyethan (absolut über LiAlH₄) werden in 50 ml Toluen vorgelegt und mit 100 mmol etherischer Methyllithium-Lösung (60 ml) versetzt. Es tritt eine leichte Trübung ein und eine Temperaturerhöhung um 2°C. Das 1,2-Dimethoxyethan wird bei Raumtemperatur im Vakuum abgezogen und der ausfallende Feststoff abfiltriert. Anschließend wird dreimalig mit 10 ml Pentan gewaschen und das Produkt im Ölpumpenvakuum bei Raumtemperatur getrocknet. Es werden 3 g weißes Pulver mit der Formel CH₃Li · [(CH₃O)₂C₂H₄]_{0,07} und einer Alkalität von 35,8 mmol/g erhalten. Der Color-I-Test, ein Test auf reaktive Lithiumalkyle nach Gilman, ist positiv. Das erhaltene Produkt brennt nicht an der Luft.

Bei Hydrolyse entwickelt sich die stöchiometrische Menge an Methan. Das 13C-NMR-Spektrum weist das signifikante Signal für eine an Lithium gebundene Methylgruppe auf, CP/MAS = - 17,0 ppm.

### Beispiel 3

11,6 g (131 mmol) Methyl-t-butylether werden in 50 ml Toluen vorgelegt und mit 66 ml (131 mmol) etherischer Methyllithium-Lösung versetzt. Es wird eine Temperaturerhöhung um 1°C beobachtet. Die Lösemittel werden bis zu einer Siedetemperatur von 60°C abdestilliert, wobei ein farbloser Feststoff anfällt, der abfiltriert und im Ölpumpenvakuum bei Raumtemperatur getrocknet wird. Man erhält 2,5 g farbloses Pulver mit einem Schüttgewicht von 0,24 g/cm³ und einer Alkalität von 34,3 mmol/g.

Der erhaltene Feststoff brennt nicht an der Luft, er entzündet sich bei Wasserzutritt. Das Produkt hat die Formel CH₃Li · (CH₃-O-C(CH₃)₃)_{0,08}.

Bei Hydrolyse entwickelt sich Methan (stöchiometrische Menge). 13C-NMR, CP/MAS = - 13,6 ppm.

### Beispiel 4

500 ml (577 mmol) Methyllithium gelöst in Diethylether werden mit 300 ml Toluen versetzt. Man erhält eine klare Lösung, aus der bis zu einer Siedetemperatur von 80°C 280 g Lösemittel abdestilliert werden. Die verbleibende Suspension wird filtriert und man erhält nach Trocknung unter Ölpumpenvakuum bei Raumtemperatur 15,7 g weißes Pulver. Das Produkt entspricht der Formel CH₃Li · [(C₂H₅)₂O]_{0,10} und ist an der Luft nicht brennbar.

### Beispiel 5 (Vergleichsbeispiel 1)

Eine Lösung von Methyllithium in Diethylether wird im Ölpumpenvakuum bis zur Trockne eingedampft. Man erhält ein weißes Pulver, welches sich bei Luftkontakt sofort entzündet.

### Beispiel 6 (Vergleichsbeispiel 2)

Man versetzt eine Diethylether-Lösung von Methyllithium mit einer bezüglich des Lithiums mehr als äquimolaren Menge Methyl-t-butylether und engt dieses Gemisch bis zur Trockne im Ölpumpenvakuum ein. Man erhält Methyllithium als weißes Pulver, welches bei Luftkontakt sofort brennt.

### Beispiel 7 (Untersuchung der Zündtemperatur)

Eine elektrische Heizplatte wird im Temperaturbereich 50, 60, 70, 80, 90, 100, 110 und 120°C alle 10°C mit ca. 0,1 g Methyllithium-Komplex mit Methyl-t-butylether (33,5 mmol/g) beaufschlagt. In keinem Fall ist ein Entzünden oder Verkohlen festzustellen.

### Beispiel 8 (Umsetzung des Produkts)

100 mmol Methyllithium-Methyl-t-butylether-Komplex der Formel CH₃Li · (CH₃-O-C(CH₃)₃)_{0,08} werden in 50 ml Toluen bei 10°C mit Acetophenon umgesetzt. Der Reaktionsverlauf ist stark exotherm. Nach hydrolytischer Aufarbeitung wird 1H-NMR-spektroskopisch 2-Phenyl-2-Propanol als Reaktionsprodukt identifiziert (CH₃ = 1,58 ppm).

### Beispiel 9 (Löslichkeitstest der Produkte)

a) 100 mmol Methyllithium-Methyl-t-butylether-Komplex der Formel CH₃Li · (CH₃-O-C(CH₃)₃)_{0,08} werden in 50 ml Toluen 1 h bei Raumtemperatur gerührt. Die Suspension wird filtriert. Das klare Filtrat zeigt keine Alkalität.
b) 100 mmol Methyllithium-Methyl-t-butylether-Komplex werden in 50 ml Toluen mit 100 mmol Tetrahydrofuran versetzt und 1 h bei Raumtemperatur gerührt, anschließend filtriert. Im Filtrat wird eine Alkalität von 0,029 mmol/g festgestellt.
c) 100 mmol Methyllithium-Methyl-t-butylether-Komplex werden mit 50 ml Toluen und 200 mmol Tetrahydrofuran versetzt und 1 h bei Raumtemperatur gerührt. Die Lösung besitzt eine Alkalität von 0,093 mmol/g.
d) 125 mmol Methyllithium-Methyl-t-butylether-Komplex werden mit 125 mmol Diethylether versetzt. Man erhält eine Lösung mit einer Alkalität von 0,050 mmol/g und bei Zusatz von weiteren 125 mmol Diethylether eine Lösung mit einer Alkalität von 0,087 mmol/g.

## Patentansprüche

1. Pulverförmige, nicht pyrophore Lithiumalkylverbindung der allgemeinen Formel LiCH₃ · xₙ, bei der x = ein Ether sowie n < 1 ist.

2. Pulverförmige, nicht pyrophore Lithiumalkylverbindung, hergestellt durch Verdünnen einer Lösung von Methyllithium in Diethylether mit einem Kohlenwasserstoff um 10 bis 50 Vol.% und Verdampfung der Lösemittel bis zur Ausfällung der Lithiumalkylverbindung.

3. Pulverförmige, nicht pyrophore Lithiumalkylverbindung nach Anspruch 1 oder 2, hergestellt durch zusätzliche Zugabe eines oberhalb 50°C siedenden aliphatischen Ethers mit mindestens 5 C-Atomen.

4. Pulverförmige, nicht pyrophore Lithiumalkylverbindung nach Anspruch 3, bei dem der zusätzlich zugegebene, oberhalb 50°C siedende Ether Methyl-t-butylether, Di-n-butylether, Diisopropylether oder 1,2-Dimethoxyethan ist.

5. Verfahren zur Herstellung einer pulverförmigen, nicht pyrophoren Lithiumalkylverbindung, gekennzeichnet durch Verdünnen einer gesättigten Lösung von Methyllithium in Diethylether mit einem aromatischen Kohlenwasserstoff um 10 bis 50 Vol.% und Verdampfung der Lösemittel bis zum Ausfallen der Lithiumalkylverbindung.

6. Verfahren nach Anspruch 5, gekennzeichnet durch die Verwendung von Benzen, Toluen, Xylen oder ihren Mischungen als aromatischer Kohlenwasserstoff zur Verdünnung der Diethylether-Lösung des Methyllithiums.

7. Verfahren nach den Ansprüchen 5 und 6, gekennzeichnet durch die zusätzliche Zugabe eines aliphatischen Ethers mit mindestens 5 C-Atomen zur Lösung des Methyllithiums im Diethylether-Kohlenwasserstoff-Gemisch.

8. Verfahren nach Anspruch 7, gekennzeichnet durch die zusätzliche Zugabe von Methyl-t-butylether, Di-n-butylether, Diisopropylether oder 1,2-Dimethoxyethan.

9. Verfahren nach einem oder mehreren der Ansprüche 5 bis 8, gekennzeichnet durch die Verdampfung der Lösemittel bei reduziertem Druck.

10. Verfahren nach einem oder mehreren der Ansprüche 5 bis 9, gekennzeichnet durch Abfiltration, Waschung mit inertem Lösemittel und Trocknung der Lithiumalkylverbindung im Vakuum.

11. Verwendung der Lithiumalkylverbindung nach einem oder mehreren der Ansprüche 1 bis 10 als Synthesemittel für die Durchführung lithiumorganischer Metallierungs- und/oder Alkylierungsreaktionen.
